# EUROPEAN PATENT SPECIFICATION

(11) **EP 4 635 413 B1**
(45) Date of publication and mention of the grant of the patent: **28.10.2026**
(21) Application number: 25169495.6
(22) Date of filing: 09.04.2025
(51) Int. Cl.: A61B 5/0235

(54) **EXHAUST PASSAGE STRUCTURE AND DIAPHRAGM PUMP**
ABGASROHRSTRUKTUR UND MEMBRANPUMPE
STRUCTURE DE PASSAGE D'ÉCHAPPEMENT ET POMPE À MEMBRANE

(30) Priority: 15.04.2024 JP 2024065428
(43) Date of publication of application: 22.10.2025
(73) Proprietor: Mabuchi Motor Oken Co., Ltd., Tokyo 206-0812 (JP)
(72) Inventor: ITAHARA, Kazuki, Tokyo, 206-0812 (JP)
(74) Representative: Samson & Partner Patentanwälte mbB

(56) References cited:
- JP-A- 2021 143 647
- JP-B2- 7 449 100

## Description

### TECHNICAL FIELD

The present invention relates to an exhaust passage structure having an exhaust passage that is opened and closed by a differential pressure valve.

### BACKGROUND ART

For example, JP2021-143647A (Document 1) discloses an exhaust passage structure having an exhaust passage that is opened and closed by a differential pressure valve. Document 1 discloses a diaphragm pump that supplies air to a cuff of an electronic sphygmomanometer. The diaphragm pump has an exhaust valve for exhausting air from an air passage between the pump and the cuff when stopped. The exhaust valve has a structure that opens the exhaust passage by lowering the discharge pressure of the pump. The exhaust passage is formed by a through hole that penetrates the outer wall of the pump. The exhaust passage is formed such that the passage cross-sectional area is constant from the upstream end to the downstream end.

In the exhaust passage structure described above, there is a problem that when the exhaust valve opens such that high-pressure air is exhausted through the exhaust passage, there are cases where high-pitched abnormal sounds similar to blow sounds are generated. Such abnormal noises can be eliminated to some extent by reducing a passage cross-sectional area of the exhaust passage. However, if the passage cross-sectional area is reduced to an extent that no abnormal noises are generated, it would not be practical since the exhaust performance is poor.

### SUMMARY OF THE INVENTION

The present invention aims to provide an exhaust passage structure and a diaphragm pump that are capable of preventing abnormal sounds, such as blow sounds, from the exhaust passage, without compromising practical exhaust performance.

One aspect of the present invention relates to an exhaust passage structure, comprising: a pressure chamber to which an inflow passage and an outflow passage are opened, a pressurized fluid flowing into the inflow passage, the fluid flowing out of the outflow passage; a diaphragm arranged in the pressure chamber, the diaphragm being configured to separate the pressure chamber into an upstream fluid chamber to which the inflow passage is opened and a downstream fluid chamber to which the outflow passage is opened; a check valve configured to flow the fluid from the upstream fluid chamber to the downstream fluid chamber; a first exhaust passage provided between the upstream fluid chamber and an exterior of the pressure chamber, the first exhaust passage having a passage cross-sectional area smaller than a passage cross-sectional area of the outflow passage, and the first exhaust passage being configured to discharge the fluid from the upstream fluid chamber to the exterior of the pressure chamber; and a second exhaust passage provided between the downstream fluid chamber and the exterior of the pressure chamber, the second exhaust passage being configured to discharge the fluid from the downstream fluid chamber to the exterior of the pressure chamber, wherein an inner surface of a wall forming the downstream fluid chamber includes a valve seat to which the second exhaust passage is opened, the diaphragm includes a valve body configured to be seated on the valve seat to close the second exhaust passage, the valve seat and the diaphragm constitute a differential pressure valve configured to close the second exhaust passage when a pressure in the upstream fluid chamber is higher than a pressure in the downstream fluid chamber, and to open the second exhaust passage when the pressure in the upstream fluid chamber is less than or equal to the pressure in the downstream fluid chamber, the second exhaust passage includes a downstream portion opened to the exterior of the pressure chamber and an upstream portion opened to the valve seat, and the upstream portion has a passage cross-sectional area larger than a passage cross-sectional area of the downstream portion.

### BRIEF DESCRIPTION OF THE DRAWINGS

FIG. 1 depicts a cross-sectional view of an exhaust passage structure according to an embodiment, with the differential pressure valve being opened.
FIG. 2 depicts a cross-sectional view of the exhaust passage structure according to an embodiment, with the differential pressure valve being closed.
FIG. 3 depicts a schematic diagram for explaining the pressure around the differential pressure valve when the passage cross-sectional area of the exhaust passage is relatively small.
FIG. 4 depicts a schematic diagram for explaining the pressure around the differential pressure valve when the passage cross-sectional area of the exhaust passage is relatively large.
FIG. 5 depicts a graph showing changes in the differential pressure on the differential pressure valve.
FIG. 6 depicts a cross-sectional view of a diaphragm pump according to another embodiment.

### DETAILED DESCRIPTION OF THE INVENTION

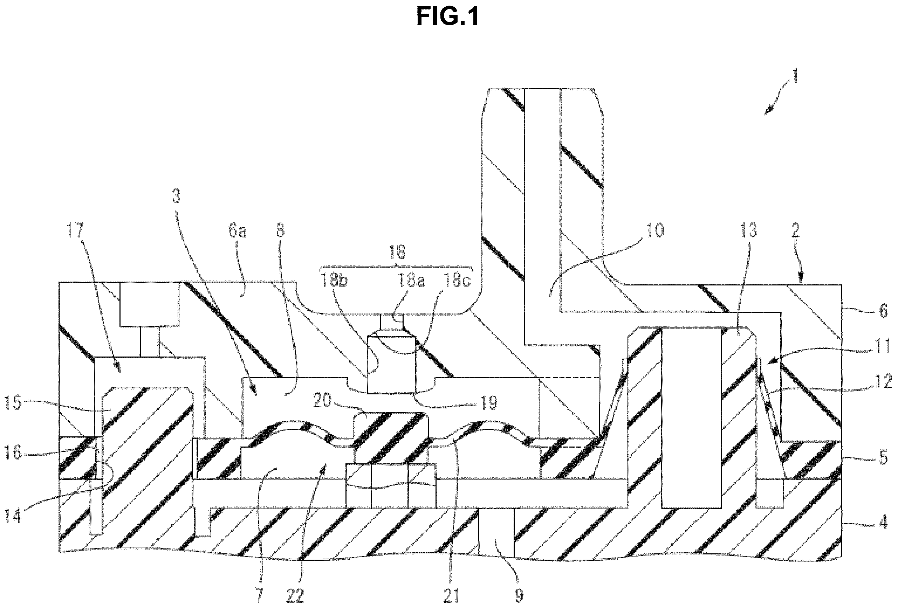

An exhaust passage structure according to an embodiment of the present invention will be described in detail with reference to FIGS. 1-5.

The exhaust passage structure 1 shown in FIG. 1 is for discharging fluid constituted by gas from a pressure chamber 3 formed by a housing 2. The housing 2 includes a case 4 (plate) illustrated at the bottom in FIG. 1, and a cover 6 layered over the case 4 via a diaphragm 5 described later. The pressure chamber 3 is separated into two chambers by the diaphragm 5 arranged in the pressure chamber 3. The two chambers are an upstream fluid chamber 7 located on the lower side of the diaphragm 5, and a downstream fluid chamber 8 located on the upper side of the diaphragm 5.

An inflow passage 9, through which the pressurized fluid flows in, is opened to the upstream fluid chamber 7. An outflow passage 10, through which the fluid flows out, is opened to the downstream fluid chamber 8. The outflow passage 10 is connected to an object-to-be-pressurized not shown.

A tapered tubular portion 12 is formed on the right end of the diaphragm 5 in FIG. 1. The tubular portion 12 is a valve body of a check valve 11. The check valve 11 is configured to flow the fluid from the upstream fluid chamber 7 to the downstream fluid chamber 8. The tip of the tubular portion 12 is freely in contact with the outer peripheral surface of the tubular body 13 convexly formed on the case 4.

A through hole 14 is formed on the left end of the diaphragm 5 in FIG. 1. A cylindrical body 15 convexly formed on the case 4 is inserted into the through hole 14. A narrow annular space 16, having a dimension allowing a slight flow of fluid, is formed between the through hole 14 and the cylindrical body 15. This space 16 constitutes a part of a small-scale exhaust passage 17 (first exhaust passage) provided between the upstream fluid chamber 7 and the exterior of the pressure chamber 3. The small-scale exhaust passage 17 discharges fluid from the upstream fluid chamber 7 to the exterior of the pressure chamber 3.

An exhaust passage 18 (second exhaust passage) communicating inside and outside of the downstream fluid chamber 8 is formed on the cover 6. The exhaust passage 18 is provided between the downstream fluid chamber 8 and the exterior of the pressure chamber 3, and discharges fluid from the downstream fluid chamber 8 to the exterior of the pressure chamber 3.

The cover 6 includes a wall 6a forming the downstream fluid chamber 8. A valve seat 19 is formed on the inner surface of the wall 6a. An upstream end, on the downstream fluid chamber 8 side, of the exhaust passage 18 is opened to the valve seat 19.

The diaphragm 5 described above includes: a valve body 20 that sits on the valve seat 19 to close the exhaust passage 18; and a thin portion 21 that supports the valve body 20.

The diaphragm 5 and the valve seat 19 constitute a differential pressure valve 22 that closes the exhaust passage 18 when the pressure in the upstream fluid chamber 7 is higher than the pressure in the downstream fluid chamber 8, and opens the exhaust passage 18 when the pressure in the upstream fluid chamber 7 is less than or equal to the pressure in the downstream fluid chamber 8.

The exhaust passage 18 is formed by a hole having a circular passage cross-sectional shape. The exhaust passage 18 includes: a downstream portion 18a opened to the exterior of the pressure chamber 3; an upstream portion 18b opened to the valve seat 19; and a boundary portion 18c between the downstream portion 18a and the upstream portion 18b. The passage cross-sectional area of the upstream portion 18b is greater than that of the downstream portion 18a. A passage cross-sectional area indicates a cross-sectional area, of a passage, perpendicular to the direction in which fluid flows. The reason for such difference in the passage cross-sectional area between the upstream portion 18b and the downstream portion 18a is that abnormal sounds similar to blow sounds are not generated by forming the exhaust passage 18 in this way. The reason why abnormal noises are not generated will be explained later.

The passage cross-sectional area of the downstream portion 18a of the exhaust passage 18 is constant from the upstream end to the downstream end in the direction in which the fluid flows. The passage cross-sectional area of the upstream portion 18b of the exhaust passage 18 is also constant from the upstream end to the downstream end. In the flow direction of the fluid, the length of the downstream portion 18a is shorter than that of the upstream portion 18b.

Meanwhile, the boundary portion 18c of the exhaust passage 18 is formed in a tapered manner such that the passage cross-sectional area gradually decreases (hole diameter decreases) from the upstream portion 18b toward the downstream portion 18a.

The passage cross-sectional area (hole diameter) of the downstream portion 18a of the exhaust passage 18 is set to be as small as possible in accordance with the decompression rate. The decompression rate herein indicates the rate at which the pressure in the downstream fluid chamber 8 decreases in the process of the closed differential pressure valve 22 being opened to discharge the fluid from the downstream fluid chamber 8. In this embodiment, the passage cross-sectional area of the downstream portion 18a of the exhaust passage 18 is set such that practical exhaust performance can be obtained in a fluid pressure device employing the exhaust passage structure 1. In other words, the downstream portion 18a has a passage cross-sectional area comparable to that of a conventional exhaust passage where sounds similar to blow sounds are generated.

Next, the operation of the exhaust passage structure 1 including the differential pressure valve 22 configured as described above will be described. As shown in FIG. 1, when the pressurized fluid flows into the upstream fluid chamber 7 from the inflow passage 9 while the differential pressure valve 22 is open and the pressure in the downstream fluid chamber 8 had become, for example, atmospheric pressure, the pressure in the upstream fluid chamber 7 rises, and a part of the fluid is discharged from the small-scale exhaust passage 17 while all the remaining fluid flows into the downstream fluid chamber 8 through the check valve 11.

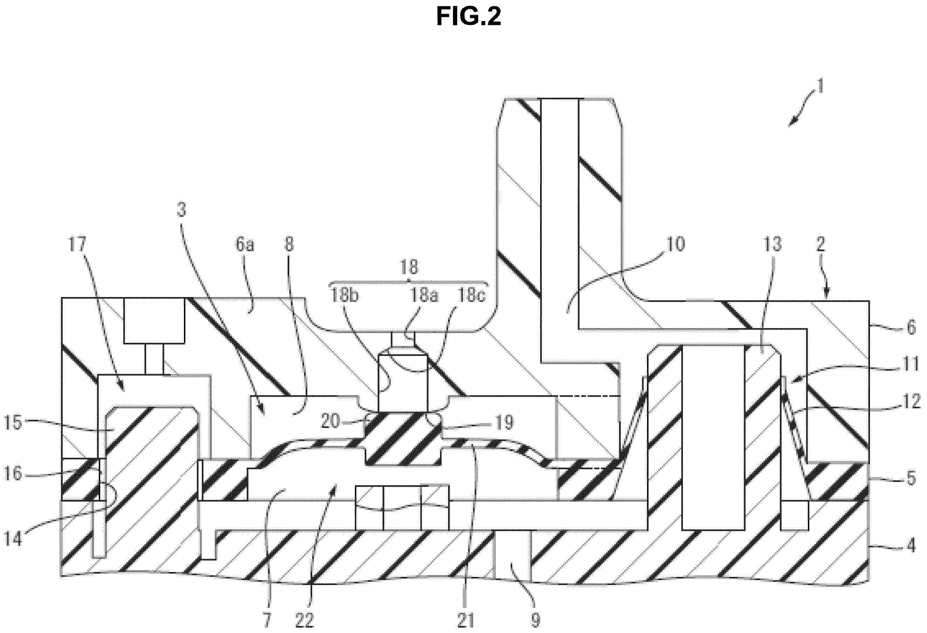

Then, when the amount of fluid flowing into the upstream fluid chamber 7 increases and the pressure in the upstream fluid chamber 7 increases, the diaphragm 5 deforms such that the valve body 20 sits on the valve seat 19, closing the exhaust passage 18, as shown in FIG. 2. In the state in which the exhaust passage 18 is thus closed, the fluid is sent from the downstream fluid chamber 8 to an object-to-be-pressurized through the outflow passage 10.

Meanwhile, when the inflow of fluid from the inflow passage 9 is stopped in the state shown in FIG. 2, the pressure in the upstream fluid chamber 7 decreases faster than that in the downstream fluid chamber 8 because the fluid in the upstream fluid chamber 7 continues to be discharged slightly from the small-scale exhaust passage 17.

Then, when the pressure in the upstream fluid chamber 7 becomes less than or equal to that in the downstream fluid chamber 8, the differential pressure valve 22 opens as shown in FIG. 1. By opening the differential pressure valve 22 in this way, the fluid is discharged from the downstream fluid chamber 8 to the exterior of the pressure chamber 3 through the exhaust passage 18. Since the exhaust passage 18 is formed such that the passage cross-sectional area of the upstream portion 18b is larger than that of the downstream portion 18a, abnormal sounds similar to blow sounds generated in conventional exhaust passages are not generated.

There are two possible reasons why such abnormal noises are not generated.

The first reason is that when the fluid flows through the exhaust passage 18, the downstream portion 18a acts substantially as a "throttle" such that the flow rate of the fluid flowing through the exhaust passage 18 is lowly suppressed. When the fluid flows through the exhaust passage 18 at high speed, a turbulent flow is generated such that a force, such as a force sucking the valve body 20 onto the valve seat 19, acts on the valve body 20. When such a force is generated, the valve body 20 repeatedly oscillates to engage and disengage from the valve seat 19 when the fluid is being evacuated, causing abnormal noises. However, by keeping the flow velocity low as in this embodiment, such forces are less likely to be generated, allowing to prevent abnormal noises.

The second reason is that the valve body 20 moves greatly to the open side when the differential pressure valve 22 opens. If the moving amount of the valve body 20 of the differential pressure valve 22 when it opens is small, the distance between the valve body 20 and the valve seat 19 is small such that there are cases where the pressure (opening force) in the downstream fluid chamber 8 and the pressure (closing force) in the upstream fluid chamber 7 are close to each other, causing the valve body 20 to repeatedly vibrate at high speed, resulting in abnormal sounds like blow sounds.

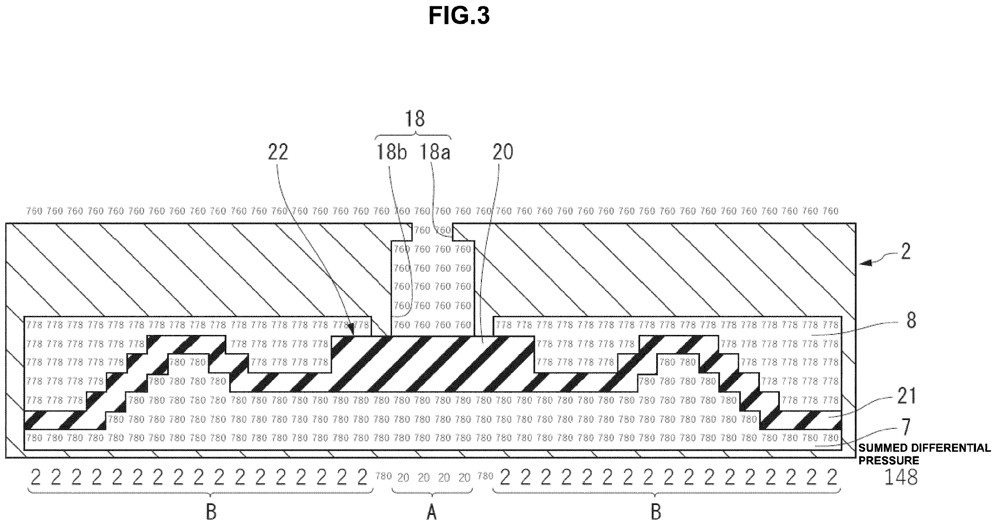

Here, the principle in which the valve body 20 moves greatly to the open side when the differential pressure valve 22 opens will be explained with reference to FIGS. 3-5. In these figures, the same or equivalent members as those described in FIGS. 1 and 2 are assigned the same sign, and the detailed descriptions thereof are omitted as appropriate.

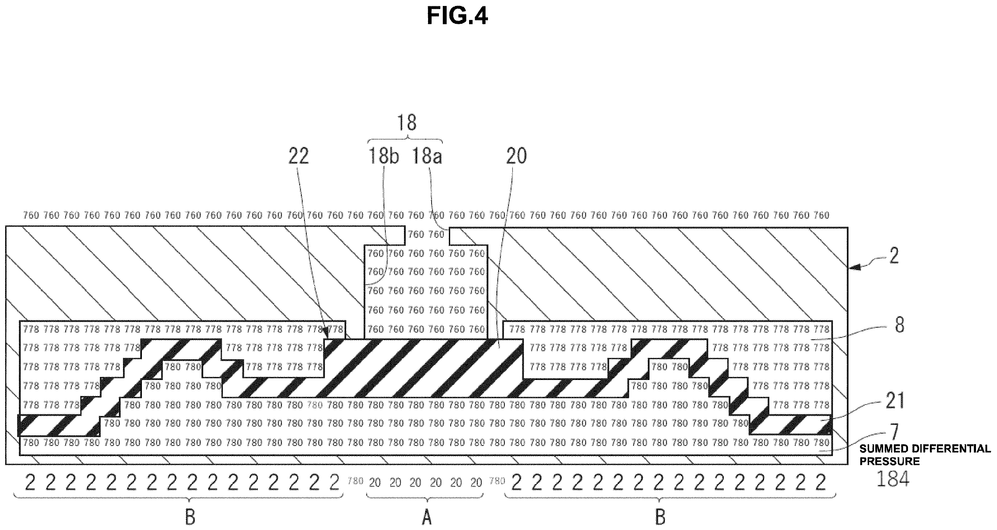

FIGS. 3 and 4 depict schematic diagrams showing a numerical example of pressures in the respective parts at the moment when the differential pressure valve 22 is closed and the supply of fluid to the upstream fluid chamber 7 is stopped, that is, at the moment when the pressurization is stopped. The only difference between FIGS. 3 and 4 is the passage cross-sectional area of the upstream portion 18b of the exhaust passage 18. FIG. 3 depicts a case where the passage cross-sectional area of the upstream portion 18b is relatively small, and FIG. 4 depicts a case where the passage cross-sectional area of the upstream portion 18b is relatively large. In the following, the differential pressure valve 22 arranged on the exhaust passage 18 illustrated in FIG. 3 is referred to as a "valve having a small hole diameter", and the differential pressure valve 22 arranged on the exhaust passage 18 illustrated in FIG. 4 is referred to as a "valve having a large hole diameter". The atmospheric pressure is assumed to be 760 mmHg, and the pressure values in the upstream fluid chamber 7, the downstream fluid chamber 8, and the exhaust passage 18 are set so that largeness and smallness of differential pressures are easy to understand in relation to this atmospheric pressure.

In FIGS. 3 and 4, the pressure in the exhaust passage 18 is at the atmospheric pressure due to atmospheric release, and the pressure on the upstream fluid chamber 7 side is at the maximum pressure. The differential pressure in a region "A" where the valve body 20 and the exhaust passage 18 overlap can be obtained by a formula, "maximum pressure in upstream fluid chamber 7" - "atmospheric pressure". The differential pressure in the outer periphery portion and the thin portion 21 on the outer side of the valve seat 19 of the valve body 20 (region "B" shown in FIGS. 3 and 4) equals to the differential pressure caused by the valve resistance (the pressure loss caused by the check valve 11) of the differential pressure valve 22 separating the front side and back side of the diaphragm 5, and is "2" in this embodiment.

In FIGS. 3 and 4, pressures in the respective portions when the pressurization is stopped are as follows.
· Pressure in exhaust passage 18: 760 mmHg
· Pressure in upstream fluid chamber 7: 780 mmHg
· Pressure in downstream fluid chamber 8: 778 mmHg

In this embodiment, the valve body 20 and the thin portion 21 are combined and simply referred to as the "valve portion". The differential pressure ((force in the closing direction) - (force in the opening direction)) between the force that pushes the valve portion in the closing direction (upward in the figure) and the force that pushes the valve portion in the opening direction (downward in the figure), that is, the difference between the pressure applied to the front side of the valve portion and the pressure applied to the back side of the valve portion, is referred to as the "summed differential pressure".

As shown in FIG. 3, the summed differential pressure on the valve having a small hole diameter is 148, and as shown in FIG. 4, the summed differential pressure on the valve having a large hole diameter is 184. Such difference in numerical values is attributed to the size of the passage cross-sectional area of the upstream portion 18b of the exhaust passage 18.

The differential pressure valve 22 begins to open when the summed differential pressure becomes negative. The pressure in the upstream fluid chamber 7 gradually decreases because the fluid continues to be discharged slightly from the small-scale exhaust passage 17. The pressure in the downstream fluid chamber 8 gradually decreases due to reasons such as a leak on the side of the object-to-be-pressurized or a leak on the check valve 11. Since the atmospheric pressure is constant, the force that pushes the valve body 20 in the opening direction becomes superior over time, and the value of the summed differential pressure eventually becomes negative.

The valve having a small hole diameter opens when the value 148 of the summed differential pressure had gradually decreased and becomes negative after the pressurization is stopped. The valve having a large hole diameter opens when the value 184 of the summed differential pressure had gradually decreased and becomes negative after the pressurization is stopped.

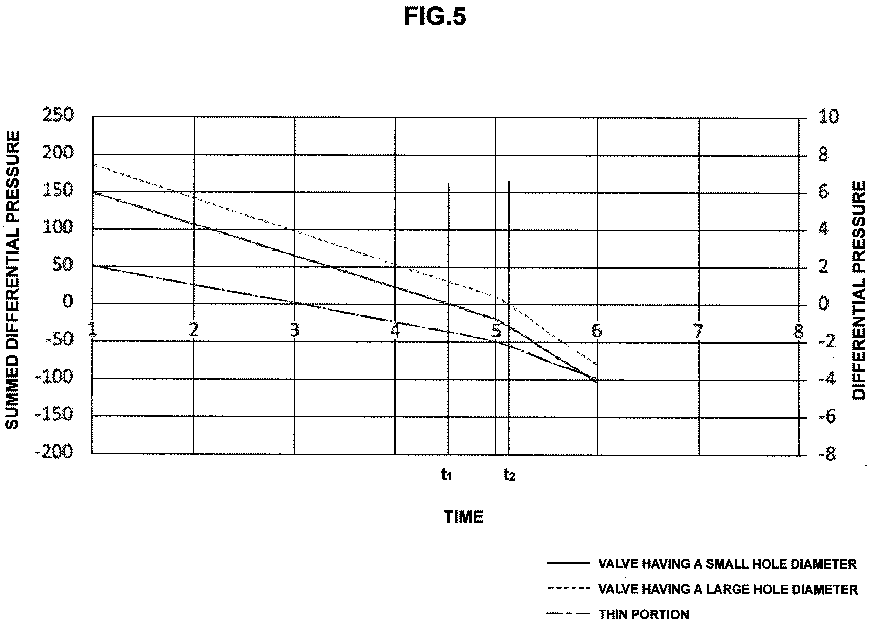

The value of the summed differential pressure varies as shown in the graph shown in FIG. 5. In the graph shown in FIG. 5, the abscissa indicates the elapsed time after the pressurization is stopped, the ordinate on the left indicates the summed differential pressure, and the ordinate on the right indicates the differential pressure. In FIG. 5, a solid line indicates changes in the summed differential pressure on the valve having a small hole diameter, and a dashed line indicates changes in the summed differential pressure on the valve having a large hole diameter. Moreover, in FIG. 5, a dashed-dot line indicates changes in the summed differential pressure on the thin portion 21 of the diaphragm 5.

As shown in FIG. 5, the summed differential pressure on the valve having a small hole diameter gradually decreases after the pressurization is stopped and becomes 0 when the time t1 = 4.5. The valve having a small hole diameter begins to open from this time. The summed differential pressure on the thin portion 21 at this time is -37 (the differential pressure is -1.5).

The summed differential pressure on a valve having a large hole diameter gradually decreases after the pressurization is stopped and becomes 0 when the time t2 = 5.1. The valve having a large hole diameter begins to open from this time. The summed differential pressure on the thin portion 21 at this time is -53 (the differential pressure is -2.1). The greater the negative magnitude of the summed differential pressure on the thin portion 21, the greater the force to push the thin portion 21 to the lower side of the figure by the differential pressure such that the valve body 20 is strongly pushed to the lower side (opening direction) at the moment it opens. When the thin portion 21 is pressed strongly in this way, it is considered that the differential pressure valve 22 is easy to open.

That is, the differential pressure on the thin portion 21 when the valve having a small hole diameter opens is -1.5, whereas the differential pressure on the thin portion 21 when the valve having a large hole diameter opens is -2.1. Therefore, the force that the thin portion 21 assists the valve body 20 in the opening direction when the valve opens is greater for a valve having a large hole diameter than for a valve having a small hole diameter.

When the passage cross-sectional area of the upstream portion 18b of the exhaust passage 18 is small as in the valve having a small hole diameter, the opening amount of the valve body 20 is relatively small when the valve is opened, and there are cases where the force of opening the valve body 20 and the force of sucking and pulling the valve body 20 onto the valve seat 19 are close, and therefore the valve body 20 vibrates at a high frequency, causing abnormal sounds similar to blow sounds. However, in a valve having a large hole diameter, the valve body 20 opens relatively widely when the valve is opened, and even if fluid flows into the exhaust passage 18 at a high rate, the force that sucks and pulls the valve body 20 onto the valve seat 19 is reduced, and therefore abnormal noises are not generated.

Therefore, in the exhaust passage structure 1 according to this embodiment, since the passage cross-sectional area of the upstream portion 18b of the exhaust passage 18 is larger than that of the downstream portion 18a, it is possible to prevent abnormal noises, such as blow sounds, from the exhaust passage 18 without compromising practical exhaust performance.

**In** the exhaust passage structure 1 according to this embodiment, the passage cross-sectional area of the downstream portion 18a of the exhaust passage 18 is constant, and the passage cross-sectional area of the upstream portion 18b of the exhaust passage 18 is constant. Therefore, the resistance when the fluid flows through the exhaust passage 18 can be reduced as much as possible, and therefore the pressure reduction rate when the differential pressure valve 22 is opened can be increased while preventing abnormal noise.

In the exhaust passage structure 1 according to this embodiment, the boundary portion 18c between the downstream portion 18a and the upstream portion 18b of the exhaust passage 18 is formed in a tapered shape such that the passage cross-sectional area gradually becomes smaller from the downstream portion 18a to the upstream portion 18b. Therefore, it is possible to prevent abnormal noises, such as wind noises, from occurring, when fluid flows from the upstream portion 18b to the downstream portion 18a.

### Diaphragm pump

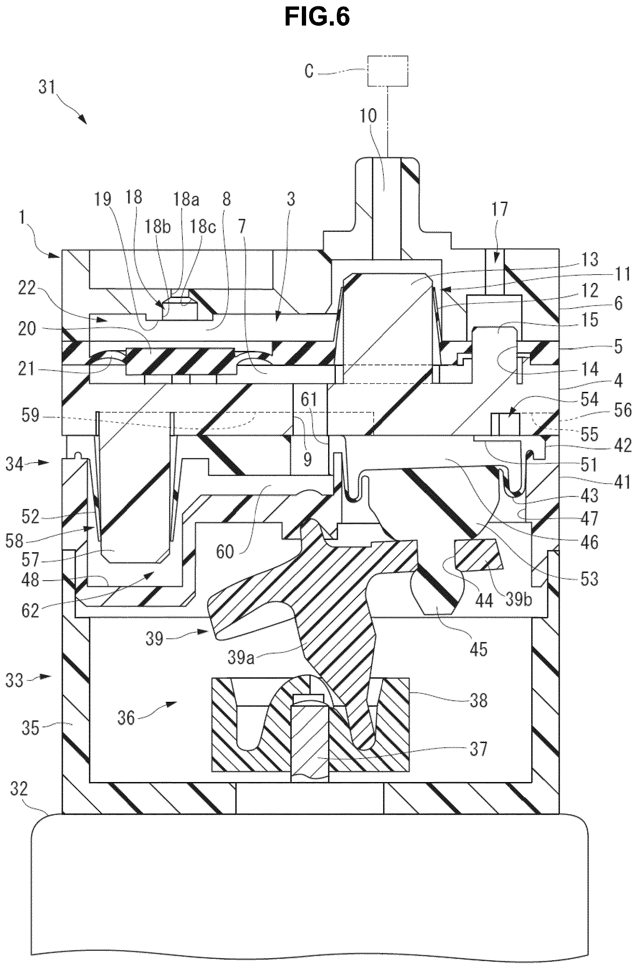

The exhaust passage structure 1 described above can be incorporated into a diaphragm pump 31 as shown in FIG. 6. In FIG. 6, the same or equivalent members as described in FIGS. 1-5. are assigned the same sign, and detailed descriptions thereof are omitted.

The diaphragm pump 31 supplies air through the exhaust passage structure 1 described above to, for example, a cuff C of an electronic sphygmomanometer (not shown), serving as an object-to-be-pressurized. The diaphragm pump 31 includes an exhaust passage structure 1 as a constituting component. The diaphragm pump 31 is mounted on a pump driving motor 32 located at the bottom of FIG. 6, and further includes: a driving unit 33 fixed onto the pump driving motor 32; and a valve portion 34 attached to the driving unit 33.

### Driving unit

The driving unit 33 includes: a first housing 35 fixed onto the pump driving motor 32; and a driving mechanism 36 (driving device) accommodated in the first housing 35. The first housing 35 is formed in the shape of a cylinder having a bottom and is fixed onto the pump driving motor 32 by bolts for fixation, not illustrated. For example, the driving mechanism 36 includes: a crank body 38 attached to a rotation axis 37 of the pump driving motor 32; and a driving body 39 connected to the crank body 38.

The driving body 39 includes: a shaft portion 39a including a lower end (first end) that engages with the crank body 38; and a plurality of arm portions 39b protruding radially outward from an intermediate portion of the shaft portion 39a. The shaft portion 39a is inclined in a predetermined direction with respect to the rotation axis 37. The lower end of the shaft portion 39a engages the crank body 38 in a manner free to rotate and oscillate so as to be able to rotate about the rotation axis 37 together with the crank body 38. The upper end (second end) of the shaft portion 39a is supported by the second housing 41 attached to the opening of the first housing 35 in a manner free to oscillate.

The arm portion 39b is provided for each of the plurality of pump portions 43 of the pump diaphragm 42 described later, and extends radially outward from the shaft portion 39a in the radial direction. Although only one arm portion 39b is illustrated in FIG. 6, there actually are as many arm portions 39b as the pump portions 43. A through hole 44 is formed on the arm portion 39b. A connecting piece 45 of the pump diaphragm 42 is engaged in the through hole 44. The connecting piece 45 is fixed onto the arm portion 39b in a state in which it penetrates the arm portion 39b.

According to the driving mechanism 36, a rotational force is applied from the rotation axis 37 of the pump driving motor 32 to rotate the crank body 38, causing the driving body 39 to swing, and the pump portion 43 of the pump diaphragm 42 to repeatedly contract and expand. That is, the driving mechanism 36 converts the rotation of the crank body 38 into reciprocating motion and increases and decreases the volume of the pump chamber 46 in the pump portion 43.

### Valve portion

For example, the valve portion 34 includes: a pump diaphragm 42 connected to the driving body 39; a second housing 41 attached to the opening portion of the first housing 35; and a case 4 of the exhaust passage structure 1. The pump diaphragm 42 is sandwiched between the second housing 41 and the case 4. The first housing 35, the second housing 41, the case 4, and the cover 6 of the exhaust passage structure 1 are circular when viewed from the axial direction of the motor 32.

The second housing 41 is formed in a cylindrical shape that is connectable to the first housing 35. The second housing 41 includes: a cylinder hole 47 for each pump portion 43 into which cylinder hole 47 the pump portion 43 of the pump diaphragm 42 described later is inserted; and a recess 48 opened toward the pump diaphragm 42.

### Pump diaphragm

The pump diaphragm 42 includes: a cup-shaped pump portion 43 opened toward the case 4; a plate-shaped suction valve 51 that protrudes from the opening portion of the pump portion 43 to the inside of the pump portion 43; and a cylindrical tubular valve body 52 inserted into the recess 48 on the second housing 41. Such pump portion 43, suction valve 51, and tubular valve body 52 are respectively provided at positions separating the pump diaphragm 42 into a plurality of sections in the circumferential direction of the second housing 41 that has a cylindrical shape. The pump portion 43 is inserted into a cylinder hole 47 formed on the second housing 41.

The opening portion of the pump portion 43 is occluded by the case 4. A pump chamber 46 including the pump portion 43 as a part of the wall is formed between the pump portion 43 and the case 4. At the bottom of the pump portion 43 that has a cup shape, there is a piston 53 and there is a connecting piece 45 that protrudes in an opposite direction to the pump chamber 46. The coupling piece 45 is coupled to the driving body 39 of the driving mechanism 36 as described above. As a result, the bottom (piston 53) of the pump portion 43 engages and disengages from the case 4 due to the swinging motion of the driving body 39 such that the volume of the pump chamber 46 is increased and decreased.

The suction valve 51 opens and closes the suction passage 54 formed on the case 4. The suction passage 54 is constituted by a first groove 55 formed on a mating surface with the pump diaphragm 42 and the second housing 41, in the case 4. The first end of the suction passage 54 is connected to the pump chamber 46 through the suction valve 51, and the second end of the suction passage 54 is connected to the fluid inlet 56 opened to the outer surface of the case 4 (the exterior of the diaphragm pump 31). The suction valve 51 opens and closes the suction passage 54 such that the atmosphere flows from the fluid inlet 56 toward the pump portion 43 in a stroke in which the pump portion 43 of the pump diaphragm 42 expands.

The tubular valve body 52 constitutes a discharge valve 58 together with a cylindrical post 57 convexly provided on the case 4. The cylindrical post 57 constitutes a valve seat of the discharge valve 58. The tubular valve body 52 is formed in a cylindrical shape and covers the outer peripheral surface of the cylindrical post 57. The discharge valve 58 is provided between a second groove 59 formed on a mating surface with the pump diaphragm 42 and the recess 48 of the second housing 41, in the case 4, and is configured so that the air (fluid) flows from the second groove 59 toward the recess 48.

The recess 48 is connected to the upstream fluid chamber 7 of the exhaust passage structure 1 through a space 60 formed between the second housing 41 and the pump diaphragm 42, a first through hole 61 formed on the pump diaphragm 42, and an inflow passage 9 formed on the case 4. The upstream fluid chamber 7 communicates with each of the plurality of pump chambers 46. The second groove 59, the space in the recess 48, the space 60, and the first through hole 61 constitute a discharge passage 62 having a first end connected to the pump chamber 46 and a second end connected to the inflow passage 9.

The case 4 of the exhaust passage structure 1 according to this embodiment is formed in a plate shape and is superimposed on the pump diaphragm 42 so as to cover the opening portion of the pump portion 43, forming a pump chamber 46 together with the pump portion 43.

### Operations

In the diaphragm pump 31 configured in this way, when the pump portion 43 of the pump diaphragm 42 expands such that the volume of the pump chamber 46 is increased, the suction valve 51 opens and the atmosphere is sucked into the pump chamber 46 through the suction passage 54. Meanwhile, when the pump portion 43 of the pump diaphragm 42 contracts such that the volume of the pump chamber 46 is decreased, air in the pump chamber 46 is sent to the upstream fluid chamber 7 through the second groove 59, the discharge valve 58, the recess 48, the space 60, the first through hole 61 and the inflow passage 9. As the volume of the pump chamber 46 decreases in this way, the upstream fluid chamber 7 is pressurized such that there is a pressure difference between the upstream fluid chamber 7 and the downstream fluid chamber 8.

As a result of the differential pressure valve 22 being closed by the pressure of air flowing into the inflow passage 9, air is supplied from the downstream fluid chamber 8 through the outflow passage 10 to, for example, a cuff C of an electronic sphygmomanometer. Thereafter, the pressure in the upstream fluid chamber 7 decreases and the differential pressure valve 22 opens while the driving mechanism 36 is stopped, thereby the air in the downstream fluid chamber 8 is evacuated through the exhaust passage 18. The diaphragm pump 31 employs the exhaust passage structure 1 shown in FIGS. 1 and 2 in the exhaust system, and therefore, despite having practical exhaust performance, abnormal sounds similar to blow sounds are not generated from the exhaust passage 18 during exhaustion.

## Claims

1. An exhaust passage structure (1), comprising:
a pressure chamber (3) to which an inflow passage (9) and an outflow passage (10) are opened, a pressurized fluid flowing into the inflow passage (9), the fluid flowing out of the outflow passage (10);
a diaphragm (5) arranged in the pressure chamber (3), the diaphragm (5) being configured to separate the pressure chamber (3) into an upstream fluid chamber (7) to which the inflow passage is opened and a downstream fluid chamber (8) to which the outflow passage is opened;
a check valve (11) configured to flow the fluid from the upstream fluid chamber (7) to the downstream fluid chamber (8);
a first exhaust passage (17) provided between the upstream fluid chamber (7) and an exterior of the pressure chamber (3), the first exhaust passage (17) having a passage cross-sectional area smaller than a passage cross-sectional area of the outflow passage (10), and the first exhaust passage (17) being configured to discharge the fluid from the upstream fluid chamber (7) to the exterior of the pressure chamber (3); and
a second exhaust passage (18) provided between the downstream fluid chamber (8) and the exterior of the pressure chamber (3), the second exhaust passage (18) being configured to discharge the fluid from the downstream fluid chamber (8) to the exterior of the pressure chamber (3), wherein
an inner surface of a wall forming the downstream fluid chamber (8) includes a valve seat (19) to which the second exhaust passage (18) is opened,
the diaphragm (5) includes a valve body (20) configured to be seated on the valve seat (19) to close the second exhaust passage (18),
the valve seat (19) and the diaphragm (5) constitute a differential pressure valve (22) configured to close the second exhaust passage (18) when a pressure in the upstream fluid chamber (7) is higher than a pressure in the downstream fluid chamber (8), and to open the second exhaust passage (18) when the pressure in the upstream fluid chamber (7) is less than or equal to the pressure in the downstream fluid chamber (8),
**characterized in that**
the second exhaust passage (18) includes a downstream portion (18a) opened to the exterior of the pressure chamber (3) and an upstream portion (18b) opened to the valve seat (19), and
the upstream portion (18b) has a passage cross-sectional area larger than a passage cross-sectional area of the downstream portion (18a).

2. The exhaust passage structure (1) according to claim 1, wherein
the passage cross-sectional area of the downstream portion (18a) of the second exhaust passage (18) is constant, and
the passage cross-sectional area of the upstream portion (18b) of the second exhaust passage (18) is constant.

3. The exhaust passage structure (1) according to claim 2, wherein
the second exhaust passage (18) further includes a boundary portion (18c) between the downstream portion (18a) and the upstream portion (18b), and
the boundary portion (18c) is formed in a tapered shape such that a passage cross-sectional area gradually decreases from the upstream portion (18b) to the downstream portion (18a).

4. The exhaust passage structure (1) according to any one of claims 1-3, wherein
the downstream portion (18a) of the second exhaust passage (18) has a length shorter than a length of the upstream portion (18b) in a direction in which the fluid flows.

5. A diaphragm pump (31), comprising:
the exhaust passage structure (1) according to any one of claims 1-4;
a pump diaphragm (42) including a cup-shaped pump portion (43);
a pump chamber (46) formed by the pump portion (43) and a plate (4) covering an opening portion of the pump portion (43);
a driving device (36) connected to a bottom of the pump portion (43), the driving device (36) being configured to transform rotation into reciprocating motion to increase and decrease volume of the pump chamber (46);
a motor (32) configured to apply a rotational force to the driving device (36);
a suction passage (54) including a first end connected to the pump chamber (46) and a second end connected to a fluid inlet (56) opened to an exterior of the diaphragm pump (31);
a suction valve (51) configured to open and close the suction passage (54) such that fluid flows from the fluid inlet (56) toward the pump chamber (46) in a stroke in which the volume of the pump chamber (46) increases;
a discharge passage (62) including a first end connected to the pump chamber (46) and a second end connected to the inflow passage (9); and
a discharge valve (58) configured to open and close the discharge passage (62) such that fluid flows from the pump chamber (46) toward the inflow passage (9) in a stroke in which the volume of the pump chamber (46) decreases.

6. The diaphragm pump (31) according to claim 5, wherein
as a result of the differential pressure valve (22) being closed due to a pressure caused by the fluid flowing through the discharge valve (58) into the inflow passage (9), the fluid is supplied from the downstream fluid chamber (8) to an object-to-be-pressurized through the outflow passage (10), and
as a result of the differential pressure valve (22) being opened due to the pressure in the upstream fluid chamber (7) being reduced while the driving device (36) is stopped, the fluid in the downstream fluid chamber (8) is discharged through the second exhaust passage (18).

## Patentansprüche

1. Abgaspassagenstruktur (1) umfassend:
eine Druckkammer (3), zu der eine Einlasspassage (9) und eine Auslasspassage (10) geöffnet sind, wobei ein unter Druck stehendes Fluid in die Einlasspassage (9) strömt und das Fluid aus dem Auslasspassage (10) austritt;
eine in der Druckkammer (3) angeordnete Membran (5), wobei die Membran (5) derart ausgebildet ist, dass sie die Druckkammer (3) in eine stromaufwärtige Fluidkammer (7), zu der der Einlasspassage m ü ndet, und eine stromabwärtige Fluidkammer (8), zu der die Auslasspassage mündet, unterteilt;
ein Rückschlagventil (11), das derart ausgebildet ist, dass es das Fluid von der stromaufwärtigen Fluidkammer (7) zur stromabwärtigen Fluidkammer (8) strömen lässt;
eine ersten Entlüftungspassage (17), die zwischen der stromaufwärtigen Fluidkammer (7) und der Außenseite der Druckkammer (3) vorgesehen ist, wobei die erste Entlüftungspassage (17) eine Passagenquerschnittsfläche aufweist, die kleiner ist als die Passagenquerschnittsfläche der Auslasspassages (10), und die erste Entlüftungspassage (17) derart ausgebildet ist, dass sie das Fluid aus der stromaufwärtigen Fluidkammer (7) nach außen aus der Druckkammer (3) ableitet; und
einen zweiten Auslasspassage (18), die zwischen der stromabwärts gelegenen Fluidkammer (8) und der Außenseite der Druckkammer (3) vorgesehen ist, wobei die zweite Auslasspassage (18) derart ausgebildet ist, dass sie das Fluid aus der stromabwärts gelegenen Fluidkammer (8) zur Außenseite der Druckkammer (3) ableitet, wobei
eine Innenfläche einer die stromabwärts gelegene Fluidkammer (8) bildenden Wand einen Ventilsitz (19) aufweist, zu dem die zweite Auslasspassage (18) hin offen ist,
die Membran (5) einen Ventilkörper (20) aufweist, der derart ausgebildet ist, dass er auf dem Ventilsitz (19) aufliegt, um den zweiten Auslasspassage (18) zu verschließen,
der Ventilsitz (19) und die Membran (5) ein Differenzdruckventil (22) bilden, das derart ausgelegt ist, dass es die zweite Auslasspassage (18) schließt, wenn der Druck in der stromaufwärtigen Fluidkammer (7) höher ist als der Druck in der stromabwärtigen Fluidkammer (8), und dass es die zweite Auslasspassage (18) öffnet, wenn der Druck in der stromaufwärtigen Fluidkammer (7) kleiner oder gleich dem Druck in der stromabwärtigen Fluidkammer (8) ist,
**dadurch gekennzeichnet, dass**
die zweite Auslasspassage (18) einen stromabwärts gelegenen Abschnitt (18a), der zur Außenseite der Druckkammer (3) hin offen ist, und einen stromaufwärts gelegenen Abschnitt (18b) umfasst, der zum Ventil -Sitz (19) hin offen ist, und
der stromaufwärtige Abschnitt (18b) eine Durchflussquerschnittsfläche aufweist, die größer ist als die Durchflussquerschnittsfläche des stromabwärtigen Abschnitts (18a).

2. Abgaspassagenstruktur (1) nach Anspruch 1, bei der
die Durchflussquerschnittsfläche des stromabwärts gelegenen Abschnitts (18a) der zweiten Abgaspassages (18) konstant ist und
die Durchflussquerschnittsfläche des stromaufwärtigen Abschnitts (18b) des zweiten Abgaspassages (18) konstant ist.

3. Abgaspassagenstruktur (1) nach Anspruch 2, bei der
die zweite Abgaspassage (18) ferner einen Grenzabschnitt (18c) zwischen dem stromabwärtigen Abschnitt (18a) und dem stromaufwärtigen Abschnitt (18b) umfasst, und
der Grenzabschnitt (18c) konisch geformt ist, sodass sich die Passagenquerschnittsfläche vom stromaufwärtigen Abschnitt (18b) zum stromabwärtigen Abschnitt (18a) hin allmählich verringert.

4. Abgaspassagenstruktur (1) nach einem der Ansprüche 1 bis 3, bei der
der stromabwärtige Abschnitt (18a) der zweiten Abgaspassage (18) in Strömungsrichtung eine kürzere Länge aufweist als der stromaufwärtige Abschnitt (18b).

5. Membranpumpe (31), umfassend:
die Abgaspassagenstruktur (1) gemäß einem der Ansprüche 1 bis 4;
eine Pumpenmembran (42) mit einem becherförmigen Pumpenabschnitt (43);
eine Pumpenkammer (46), die durch den Pumpenabschnitt (43) und eine Platte (4) gebildet wird, die einen Öffnungsabschnitt des Pumpenabschnitts (43) abdeckt;
eine Antriebsvorrichtung (36), die mit einem Boden des Pumpenabschnitts (43) verbunden ist, wobei die Antriebsvorrichtung (36) derart ausgelegt ist, dass sie eine Drehbewegung in eine Hin- und Herbewegung umwandelt, um das Volumen der Pumpenkammer (46) zu vergrößern und zu verkleinern;
einen Motor (32), der derart ausgelegt ist, dass er eine Drehkraft auf die Antriebsvorrichtung (36) ausübt;
einen Saugpassage (54) mit einem ersten Ende, das mit der Pumpenkammer (46) verbunden ist, und einem zweiten Ende, das mit einem Fluideinlass (56) verbunden ist, der zur Außenseite der Membranpumpe (31) hin offen ist;
ein Ansaugventil (51), das derart ausgebildet ist, dass es die Ansaugpassage (54) öffnet und schließt, sodass Fluid in einem Hub, in dem sich das Volumen der Pumpenkammer (46) vergrößert, vom Fluideinlass (56) in Richtung der Pumpenkammer (46) strömt;
eine Auslasspassage (62) mit einem ersten Ende, das mit der Pumpenkammer (46) verbunden ist, und einem zweiten Ende, das mit der Zuflusspassage (9) verbunden ist; und
ein Auslassventil (58), das derart ausgelegt ist, dass es den Auslasspassage (62) öffnet und schließt, sodass Fluid in einem Hub, in dem das Volumen der Pumpenkammer (46) abnimmt, von der Pumpenkammer (46) in Richtung der Zuflusspassages (9) strömt.

6. Membranpumpe (31) nach Anspruch 5, bei der
das Fluid infolge eines Schließens des Differenzdruckventils (22) - bedingt durch einen Druck, der durch das durch das Auslassventil (58) in den Einlasspassage (9) strömende Fluid verursacht wird - von der stromabwärts gelegenen Fluidkammer (8) über den Auslasspassage (10) zu einem zu beaufschlagenden Objekt geleitet wird, und
das Differenzdruckventil (22) aufgrund eines Druckabfalls in der stromaufwärtigen Fluidkammer (7) bei stillstehender Antriebsvorrichtung (36) geöffnet wird, wodurch das Fluid in der stromabwärtigen Fluidkammer (8) durch die zweite Auslasspassage (18) abgelassen wird.

## Revendications

1. Structure de passage d'échappement (1), comportant :
une chambre de pression (3) vers laquelle un passage d'entrée (9) et un passage de sortie (10) sont ouverts, un fluide sous pression s'écoulant dans le passage d'entrée (9), le fluide s'écoulant hors du passage de sortie (10) ;
une membrane (5) agencée dans la chambre de pression (3), la membrane (5) étant configurée pour séparer la chambre de pression (3) en une chambre de fluide amont (7) vers laquelle le passage d'entrée est ouvert et une chambre de fluide aval (8) vers laquelle le passage de sortie est ouvert ;
un clapet anti-retour (11) configuré pour faire circuler le fluide depuis la chambre de fluide amont (7) vers la chambre de fluide aval (8) ;
un premier passage d'échappement (17) prévu entre la chambre de fluide amont (7) et un extérieur de la chambre de pression (3), le premier passage d'échappement (17) présentant une zone de section transversale de passage inférieure à une zone de section transversale de passage du passage de sortie (10), et le premier passage d'échappement (17) étant configuré pour évacuer le fluide de la chambre de fluide amont (7) vers l'extérieur de la chambre de pression (3) ; et
un deuxième passage d'échappement (18) prévu entre la chambre de fluide aval (8) et l'extérieur de la chambre de pression (3), le deuxième passage d'échappement (18) étant configuré pour évacuer le fluide de la chambre de fluide aval (8) vers l'extérieur de la chambre de pression (3), dans laquelle
une surface interne d'une paroi formant la chambre de fluide aval (8) comprend un siège de vanne (19) vers lequel le deuxième passage d'échappement (18) est ouvert,
la membrane (5) comprend un corps de vanne (20) configuré pour être positionné sur le siège de vanne (19) pour fermer le deuxième passage d'échappement (18),
le siège de vanne (19) et la membrane (5) constituent une vanne de pression différentielle (22) configurée pour fermer le deuxième passage d'échappement (18) lorsqu'une pression dans la chambre de fluide amont (7) est supérieure à une pression dans la chambre de fluide aval (8), et pour ouvrir le deuxième passage d'échappement (18) lorsque la pression dans la chambre de fluide amont (7) est inférieure ou égale à la pression dans la chambre de fluide aval (8),
**caractérisée en ce que**
le deuxième passage d'échappement (18) comprend une partie aval (18a) ouverte vers l'extérieur de la chambre de pression (3) et une partie amont (18b) ouverte vers le siège de vanne (19), et
la partie amont (18b) présente une zone de section transversale de passage plus grande qu'une zone de section transversale de passage de la partie aval (18a).

2. Structure de passage d'échappement (1) selon la revendication 1, dans laquelle
la zone de section transversale de passage de la partie aval (18a) du deuxième passage d'échappement (18) est constante, et
la zone de section transversale de passage de la partie amont (18b) du deuxième passage d'échappement (18) est constante.

3. Structure de passage d'échappement (1) selon la revendication 2, dans laquelle
le deuxième passage d'échappement (18) comprend en outre une partie limite (18c) entre la partie aval (18a) et la partie amont (18b), et
la partie limite (18c) est formée en forme effilée de sorte qu'une zone de section transversale de passage diminue progressivement de la partie amont (18b) à la partie aval (18a).

4. Structure de passage d'échappement (1) selon l'une quelconque des revendications 1 à 3, dans laquelle
la partie aval (18a) du deuxième passage d'échappement (18) présente une longueur plus courte qu'une longueur de la partie amont (18b) dans une direction dans laquelle le fluide s'écoule.

5. Pompe à membrane (31), comportant :
la structure de passage d'échappement (1) selon l'une quelconque des revendications 1 à 4 ;
une membrane de pompe (42) comprenant une partie de pompe (43) en forme de coupelle ;
une chambre de pompe (46) formée par la partie de pompe (43) et une plaque (4) recouvrant une partie d'ouverture de la partie de pompe (43) ;
un dispositif d'entraînement (36) relié à un fond de la partie de pompe (43), le dispositif d'entraînement (36) étant configuré pour transformer la rotation en mouvement alternatif pour augmenter et diminuer le volume de la chambre de pompe (46) ;
un moteur (32) configuré pour appliquer une force de rotation au dispositif d'entraînement (36) ;
un passage d'aspiration (54) comprenant une première extrémité reliée à la chambre de pompe (46) et une deuxième extrémité reliée à une entrée de fluide (56) ouverte vers un extérieur de la pompe à membrane (31) ;
une vanne d'aspiration (51) configurée pour ouvrir et fermer le passage d'aspiration (54) de sorte que le fluide s'écoule de l'entrée de fluide (56) vers la chambre de pompe (46) dans une course dans laquelle le volume de la chambre de pompe (46) augmente ;
un passage d'évacuation (62) comprenant une première extrémité reliée à la chambre de pompe (46) et une deuxième extrémité reliée au passage d'entrée (9) ; et
une vanne d'évacuation (58) configurée pour ouvrir et fermer le passage d'évacuation (62) de sorte que le fluide s'écoule de la chambre de pompe (46) vers le passage d'entrée (9) dans une course dans laquelle le volume de la chambre de pompe (46) diminue.

6. Pompe à membrane (31) selon la revendication 5, dans laquelle
à la suite de la fermeture de la vanne de pression différentielle (22) en raison d'une pression causée par le fluide s'écoulant à travers la vanne d'évacuation (58) dans le passage d'entrée (9), le fluide est acheminé depuis la chambre de fluide aval (8) vers un objet à mettre sous pression par le passage de sortie (10), et
à la suite de l'ouverture de la vanne de pression différentielle (22) en raison de la réduction de la pression dans la chambre de fluide amont (7) pendant que le dispositif d'entraînement (36) est arrêté, le fluide dans la chambre de fluide aval (8) est évacué par le deuxième passage d'échappement (18).
